# EUROPEAN PATENT APPLICATION

(11) **EP 4 792 788 A1**
(43) Date of publication of application: **19.08.2026**
(21) Application number: 25382137.5
(22) Date of filing: 17.02.2025
(51) Int. Cl.: A61F 9/00, A61M 5/19, A61M 5/20, A61M 5/24

(54) **DEVICE FOR COMBINING INGREDIENTS OF A SUBSTANCE AND FOR DISPENSING IT IN A CONTROLLED MANNER**

(71) Applicant: Brill Engines, S.L., 08022 Barcelona (ES)
(72) Inventor: BUISAN FERRER, Josep, E-08006 Barcelona (ES); NIETO CAVIA, Laura, E-08027 Barcelona (ES)
(74) Representative: ABG Intellectual Property Law, S.L.

(57) **Abstract**

The present invention relates to a device for combining ingredients of a substance and for dispensing it in a controlled manner, comprising an enclosed space of variable volume which contains, under leak-tight conditions, a first ingredient in a fluid state; a container which contains, under leak-tight conditions, a second ingredient and which is linked to a plunger; a liquid dose dispensing group; and a first and a second barrier which originally prevent fluid communication between the enclosed space, the container, and the dispensing group, the plunger being configured such that, in a relative displacement stroke of the container with respect to the enclosed space in a loading direction, the following actions are sequentially triggered: opening of the first barrier, enabling fluid communication between the enclosed space and the container, driving of the first ingredient to the container, for the combination thereof with the second ingredient obtaining the substance, and opening of the second barrier, enabling fluid communication between the container and the dispensing group, the device being provided with a cavity that expands and shrinks into which gas dislodged from the container by the first ingredient is insufflated and from which this gas is exhaled into the container when a dose of the substance is dispensed.

## Description

### Technical Field of the Invention

The invention belongs to the field of devices and methods for combining ingredients of a substance and for dispensing the substance obtained from said combination in a controlled manner.

### Background of the Invention

There are different types of devices that allow first combining, for example, mixing, generally two ingredients of a substance and then administering or dispensing the substance obtained from said combination. These devices are designed to ensure a precise and efficient combination of the ingredients making up the substance when said substance is to be administered or dispensed and are commonly used in the administration of medicinal products, cosmetic products, and other compounds.

A specific example of devices of this type are those that allow reconstituting a lyophilisate by *in situ* mixing of the lyophilisate with a serum for reconstituting same.

Lyophilization is a process used to preserve active substances that are temperature sensitive or have a limited shelf life in liquid form. This process involves freezing the substance and then removing water by sublimation, resulting in a lyophilized powder that is much more stable and easier to store.

Lyophilization helps to prolong the shelf life of medicinal products and facilitates their transport as they do not require refrigeration.

Powder lyophilisate is usually reconstituted by adding a diluent serum (e.g., saline or sterile water) to the lyophilized powder.

Prefilled syringes are one of the common ways to administer lyophilized medicinal products.

These syringes contain both the lyophilized substance and the diluent serum in separate compartments, and users or healthcare professionals can easily reconstitute the solution by mixing the two ingredients. Some syringes allow mixing ingredients by pressing on a plunger that breaks a separation between the lyophilisate and the diluent serum. It is this same plunger that then ensures the delivery of the reconstituted substance out of the syringe, for example, for parenteral administration, without being able to reuse the substance that may remain.

In the case of ophthalmic products, for example, the reconstitution process (i.e., converting the lyophilized powder back into a liquid solution) must not only be precise, given that any error in the mixture or dosage may have a very negative effect on the efficacy of the drug substance to be administered, but the process must also be performed in sterile conditions that must also be ensured after mixing, during the subsequent storage of the drug substance to be administered from which the doses for consumption and/or application will be extracted gradually according to an associated guideline.

Those known devices, which in addition to being capable of reconstituting a lyophilisate are equipped with means for metering reconstituted lyophilisate, either contain the lyophilisate in vacuum conditions, which complicates the device manufacturing and assembly process, or comprise air intake means to make it possible for the reconstituted lyophilisate to exit the device, which then prevents maintaining the desired sterile conditions inside the device and particularly in those parts or components in contact with the reconstituted lyophilisate. Furthermore, other than the valves involved in regulating the flow of the reconstituted lyophilisate when dispensing same, the known solutions require equipping the devices with various valve elements that are, however, for regulating air intake to make it possible to dislodge same or to regulate the expulsion of air so that the reconstituted lyophilisate is dispensed without mixing with air, which is of interest, for example, for droplet metering.

An objective of the invention relates to a device, alternative to known devices, which is suitable and prepared to be able to reconstitute a lyophilisate and to be able to dispense the reconstituted lyophilisate in a controlled manner, for example, in predetermined doses.

Another objective of the invention relates to the device being versatile and being able to be used to combine ingredients of a substance and to dispense in a controlled manner, for example, in predetermined doses, the substance obtained by combining said ingredients, with both ingredients being able to be non-solid.

A further objective of the invention relates to a device which can perform these functions without taking air in from the outside and/or without expelling air to the outside, for the purpose of ensuring the environmental conditions, for example, sterile conditions, under which the ingredients to be combined are contained in the device during and after their combination inside the device.

A further objective of the invention relates to a device which is capable of achieving the other objectives and which is constructively simple; and/or which does not require complex processes for obtaining and preparing or assembling same; and which is easy to handle by a user.

### Description of the Invention

The present invention provides a solution to the aforementioned problems by means of a device according to claim 1 and a method according to claim 15. The dependent claims define preferred embodiments of the invention.

In a first inventive aspect, the invention provides a device for combining ingredients of a substance and for dispensing it in a controlled manner, wherein the device comprises an enclosed space of variable volume which contains, under leak-tight conditions, a first ingredient in a fluid state; a container which contains, under leak-tight conditions, a second ingredient in a gas atmosphere and which is linked to a plunger operating on the enclosed space; and a liquid dose dispensing group that can be actuated by a user.

The device is essentially characterized in that it further comprises a first and a second barrier which, in an original state of the device before the first use thereof, prevent fluid communication between the enclosed space, the container, and the dispensing group, the plunger being configured such that, in displacement stroke A of the container with respect to the enclosed space, in a loading direction that approaches the enclosed space, the following actions are sequentially triggered:
- opening of the first barrier, enabling fluid communication between the enclosed space and the container,
- driving of the first ingredient to the container, for the combination thereof with the second ingredient inside the container obtaining the substance, and
- opening of the second barrier, enabling fluid communication between the container, and the dispensing group,
with the device further being provided with a cavity adapted for insufflating into same the gas which is dislodged from the container by introducing the first ingredient into the container and for exhaling this gas back into the container as doses of the substance are dispensed as a result of the actuation of the dispensing group.

In the context of the present invention, the terms "combining ingredients" and "mixing ingredients" can be used interchangeably.

In the context of the present invention, the term "ingredient" is used to refer to a component the combination of which with at least another component is required to obtain the substance to be dispensed. This component can be a preparation and formed in turn by several combined or mixed elements.

In the context of the present invention, "under leak-tight conditions" should be understood as the impossibility of the passage of air or any other fluid, including gaseous fluid and particularly air vapor.

In a state of the device before the first use thereof, the device is thus prepared to contain the first ingredient and the second ingredient, physically separated from one another and isolated from the outside environment. The device is furthermore prepared so that, when the substance is needed for the first time, a user can readily carry out an operation of combining the first ingredient with the second ingredient to obtain said substance and to contain it isolated from the outside environment, for dispensing it in a controlled manner, specifically in doses.

Furthermore, the device ensures that, when the substance is needed for the first time, a user carries out the operation of combining the first ingredient with the second ingredient to obtain said substance efficiently, in the sense that the mixing of ingredients is complete.

The combination of the ingredients and the dispensing of the substance obtained with said combination are thus carried out in a leak-tight manner with respect to the outside of the device, in terms of the absence of the entry or exit of air from and to the outside of the device, respectively, neither during the operation of combining ingredients nor during the successive operations of dispensing doses of the obtained substance. The ingredients and the substance obtained from their combination are confined inside the device without coming into contact with any surface within reach of an outer atmosphere that may be contaminated.

Leak-tight conditions allow preventing, for example, oxidation of both first and second ingredients and of the obtained substance, allow continuously ensuring that the sterile conditions under which both first and second ingredients were originally contained in the device do not change upon obtaining the substance resulting from their combination, and allow preventing the wetting of both ingredients with water vapor from the outside environment, which is particularly relevant when one of the ingredients is, for example and as exemplified below, a lyophilisate.

In one embodiment and according to a constructive solution, the plunger comprises a fluid communication channel with a first end of the communication channel that opens into the container and with a second end of the communication channel that opens into the enclosed space in a first phase of stroke A and into the dispensing group in a second phase of stroke A.

In one embodiment and according to a constructive solution compatible with the preceding embodiment, the plunger comprises a respiration channel with a first end of the respiration channel that opens into the container and with a second end of the respiration channel that opens into the cavity.

In relation to the foregoing and according to an embodiment of interest, the plunger comprises a plunger rod and a plunger head, wherein the plunger rod is firmly connected to a mouth of the container and supports the fluid communication channel and the respiration channel; and wherein the plunger head has a passage for the plunger rod therethrough; the plunger rod and the plunger head being originally coupled to one another with a gap in the loading direction such that sequentially in displacement stroke A of the container, the first barrier opens due to the thrusting of the distal end of the plunger rod through the passage of the plunger head, the enclosed space shrinks due to the displacement of the plunger head, driven in movement by the plunger rod, and the second barrier opens due to the thrusting of the distal end of the plunger rod.

In one embodiment, the cavity is a cavity with the capacity to expand and shrink varying its volume by an amount at least equal to the volume of the first ingredient.

In relation to the preceding embodiment and according to an embodiment of interest, at least one wall of the cavity is a flexible wall which provides the cavity with the possibility of inflating and deflating when the gas dislodged from the container is insufflated into same and when the gas is insufflated into the container, respectively.

In relation to the preceding embodiment of interest and according to a particular embodiment, the flexible wall of the cavity is formed by a single membrane secured in a leak-tight manner to the plunger rod.

In relation to the preceding particular embodiment and according to a more particular constructive solution, the plunger rod comprises a rigid overhang with a bottom and a side wall; and the membrane has a central passage for the plunger rod, with there being determined in the membrane respective inner perimeter area and outer perimeter area secured to an annular step formation of the plunger rod and to the border of the mentioned side wall of the overhang, respectively.

In a particular embodiment of the device, the plunger rod and the plunger head comprise respective rigid parts facing one another and configured to jointly determine a housing inside which the cavity can expand and shrink.

In relation to the preceding particular embodiment and according to an embodiment of interest, since the rigid parts of the plunger head and of the overhang of the plunger rod are facing one another, this arrangement is achieved with the interposition of the inner perimeter area and outer perimeter area of the membrane, flattening said areas.

In a preferred variant, the device comprises end of stroke A stops.

In one embodiment, the second ingredient is a lyophilisate, preferably in a solid state, such as lyophilized powder; and the first ingredient is a diluent serum for the lyophilisate, preferably a serum in a liquid state. In this embodiment, the substance obtained by the combination of the first and second ingredients is obtained by mixing the mentioned first and second ingredients and is a reconstituted substance.

According to one embodiment, the container is a rigid container, particularly a vial and more particularly a vial suitable for being the same one used during preparation of the lyophilisate, for example, using known techniques for liquid extraction by freezing and sublimation.

Advantageously, and unlike other syringe-type devices, the device of the invention is particularly designed so that there is no need to change the container between the one used for the preparation of the lyophilisate and the one incorporated in the device. This simplifies the assembly of the device and allows using standard techniques and tools for the preparation of the lyophilisate in vials in use.

The invention can be put into practice with a first and a second ingredient of another nature or state, other than a liquid diluent serum and a solid lyophilisate, for example, with a first and a second ingredient both originally contained in a fluid state, for example both in a liquid state, and for obtaining a substance for ophthalmological use or for another use.

In one embodiment, the obtained substance is a liquid substance.

In one embodiment, the obtained substance is a drug substance.

In one embodiment, the obtained substance is an ophthalmic medicinal product.

In one embodiment, the obtained substance is an ophthalmic medicinal product for metering thereof in the form of droplets or in spray form.

In one embodiment, the gas contained in the container is sterile air.

In one embodiment, the first and second barriers are respective breakable seals. The invention contemplates that the first or the second barrier, or both, are tear-off seals, for example, in the form of a thin film made, for example, of a heat-sealable material, where known gas- and moisture-impermeable materials used in the packaging of pharmaceutical products, having a substantially discoidal configuration in one example, may be used.

Other ways to implement the barriers are viable in order to fulfill the purpose explained below. For example, as an alternative to breaking or tearing off, the barriers can be designed to be detached at least in part from a surface to which they are adhered in the original state of the device before the first use thereof.

According to one embodiment, the device comprises an outer casing and a support bearing the container, wherein the casing receives at one service end the fitting of the support bearing the container and wherein the dispensing group comprises at least one push button that can be actuated to promote the dispensing of a dose of the substance that is operatively coupled to the casing at one end of the casing opposite the service end, with the push button being able to adopt at least two angular positions with respect to the casing, one of which is a locking position and the other an unlocking position with the push button being able to be actuated only towards the casing and in the loading direction to promote the dispensing of a dose of the substance when it adopts the unlocking position.

In a second inventive aspect, the invention provides a method for combining ingredients of a substance and for dispensing it in a controlled manner, wherein the method comprises, with one and the same device:
- enabling fluid communication between an enclosed space of variable volume which contains, under leak-tight conditions, a first ingredient in a fluid state and a container which contains, under leak-tight conditions, a second ingredient in a gas atmosphere;
- transferring the first ingredient into the container to obtain the substance, wherein the substance results from the combination of the first and second ingredients inside the container; and
- enabling fluid communication between the container and a liquid dose dispensing group that can be actuated to dispense doses of the substance,
and wherein the method further comprises:
- insufflating gas dislodged from the container when the first ingredient is transferred into a cavity configured to exhale gas from the cavity back into the container when doses of the substance are dispensed as a result of the actuation of the dispensing group.

All the features described in this specification (including the claims, description, and drawings) can be combined with one another, with the exception of the combinations of such mutually exclusive features.

### Brief Description of the Drawings

These and other features and advantages of the invention will become more apparent based on the following detailed description of a preferred embodiment, given only by way of illustrative and non-limiting example in reference to the attached figures.
Figure 1 is an exploded view of a device which exemplifies the invention, showing the main components thereof in a correlative fitting position.
Figures 2 to 4 show the device of Figure 1 in a loading sequence to obtain a substance to be dispensed which is a combination of two ingredients, wherein each figure shows the device externally and according to a plane of section.
Figures 5a and 5b show the device of Figure 1 externally, with a push button of the substance dose dispensing group adopting different respective positions: a locked position and an unlocked position.
Figures 6a and 6b both show a section view of the device of Figure 1, with the push button of the dose dispensing group adopting respective locked and unlocked positions, the latter furthermore in an instant during the actuation thereof to dispense a dose of the substance.

### Detailed Description

The invention is exemplified by means of the device 100 of the drawings.

The specific device 100 is a device suitable for dispensing a liquid substance 6, which is obtained by means of reconstituting a lyophilisate, in a controlled manner.

To that end, the device 100 is prepared to contain, originally separated from one another and under leak-tight conditions, a first ingredient 1, in the specific case of the example a serum in a fluid state, and specifically a liquid; and a second ingredient 2, in the specific case of the example a lyophilisate in a solid state.

The device 100 is furthermore prepared to enable combining the first and second ingredients 1 and 2 in a mixture, obtaining a substance 6, in a liquid state, in the specific case of the example resulting from the reconstitution of the lyophilisate with the serum.

Furthermore, the device 100 is also prepared to enable dispensing the obtained substance 6 in a controlled manner.

The device 100 is particularly designed to keep the space containing the ingredients and the obtained substance from the outside environment, which allows preventing, for example, the entry of moisture which may cause the spoilage of the lyophilisate and/or the oxidation of the ingredients and of the obtained substance, and/or continuously ensuring that the sterile conditions under which both first and second ingredients were contained in the device do not change upon obtaining the substance resulting from the combination of the ingredients.

The specific device 100 which exemplifies the invention is sized to be easily handled with hands, specifically to enable dispensing the substance 6 for ophthalmological use in a controlled manner.

As mentioned above, the invention can be put into practice with a first and a second ingredient of another nature or state, other than a liquid serum and a solid lyophilisate.

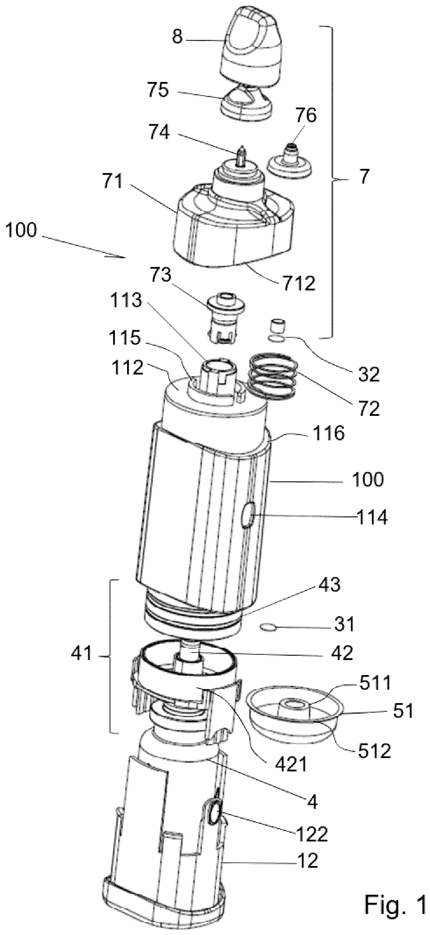

Figure 1 illustrates the main components of the device 100 according to a specific constructive solution and in a correlative assembly position, although there may be other valid constructive solutions.

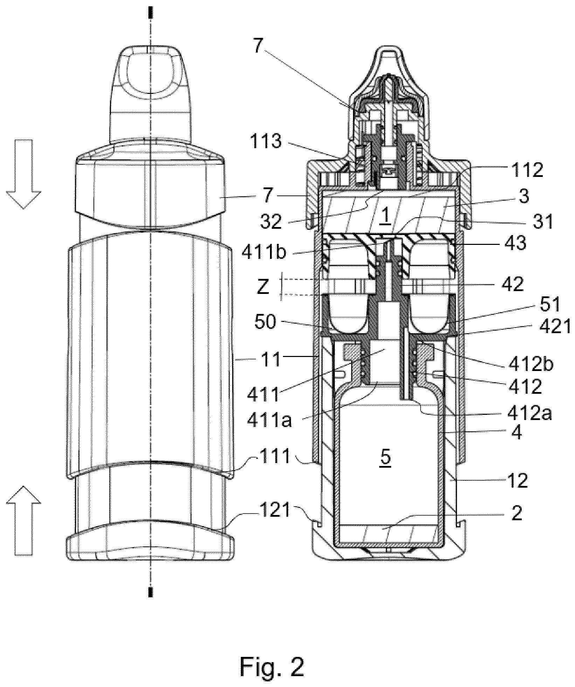

Figure 2 shows the same device 100 with the components already assembled and in an original state which this device 100 will adopt before the first use thereof.

These components and their relationship are explained below supported by these Figures 1 and 2.

The aforementioned main components comprise a main casing 11 having an essentially tubular shape into which there can be fitted, through a service end, a support 12 bearing a container 4, in the example a standard vial normalized for use in medicinal products, which stores the second ingredient 2, i.e., the lyophilisate in the example.

Particularly, the vial is the same glass vial used for the factory preparation of the lyophilisate. Advantageously, the assembly of the device 100 therefore does not require transferring the lyophilisate from the container used for its preparation to another receptacle of the device or trying to prepare the lyophilisate in receptacles made specifically for the device, which would require new tools and adaptation of standard processes.

The mentioned fitting of the support 12, bearing the container 4, is carried out by arranging another component of the dispenser 100 inside the casing 11. This other component is a plunger 41.

In the device 100 which exemplifies the invention, the plunger 41 has two parts: a plunger rod 42 and a plunger head 43.

The plunger rod 42, the functionality of which will be explained better below, is coupled to the only mouth of the container 4 and is hollow, thereby determining a fluid communication channel 411 to and from the container 4 through the mouth of the container 4. This fluid communication channel 411 has a first end 411a that opens into the container 4, a second end 411b that will open into a location that depends on the relative position of the container 4 with respect to the casing 11.

In the device 100, the plunger rod 42 has a concentric annular overhang 421 in which a bottom and a side wall are distinguished, which overhang determines a seat for a membrane 51, and the overhang 421 and the membrane 51 together will determine a chamber 50 that can expand and shrink.

In the specific exemplary device 100, as shown in Figure 1, the membrane 51 is originally in the form of a vessel, with a central passage for the plunger rod 42, determining a respective circular inner perimeter area 511 and a respective circular outer perimeter area 512 both configured to be secured in a leak-tight manner to the plunger rod 42 by heat sealing or in another suitable way, with the inner perimeter area 511 being secured to an annular step formation and the outer perimeter area 512 to the upper border of the side wall of the overhang 421, so that the chamber 50 expands without leakage when a gas is insufflated into same, specifically a gas from the container 4, and so that it can shrink when it exhales the gas back into the container 4, as will be described in greater detail below.

For this gas communication between the container 4 and the chamber 50, the plunger rod 42 of the device 100 is configured such that it comprises a respiration channel 412. A first end 412a of this respiration channel 412 opens into the container 4 and a second end 412b of this respiration channel 412 opens into the cavity 50.

In turn, the plunger head 43, by way of a piston, fits inside the casing 11 acting as a piston sleeve, determining an enclosed space 3 of variable volume, based on the instantaneous position adopted by the plunger head 43 inside the casing 11, between the plunger head 43 and a closure wall 112 which partially closes the end opposite the service end of the casing 11. There is formed in this closure wall 112 an outlet through a tube 113 for fluid connection with a dispensing group 7 described in greater detail below.

The first ingredient 1, i.e., the liquid serum in the example, is originally stored in this enclosed space 3.

Figure 1 serves to illustrate two elements, i.e., a first barrier 31 and a second barrier 32, that contribute to confining this first ingredient 1 and the second ingredient 2 in a leak-tight manner.

In the specific example, the first and second barriers 31 and 32 are both tear-off seals having a discoidal configuration for example. Suitable tear-off materials are available on the market. One example is the material marketed under the registered trademark PierceASeal Foil^{™}. However, other solutions are viable to fulfill the purpose explained below.

First, other features of the device 100 that will be understood better based on Figure 2 showing the device 100 in its original state, before the first use thereof, are described in detail.

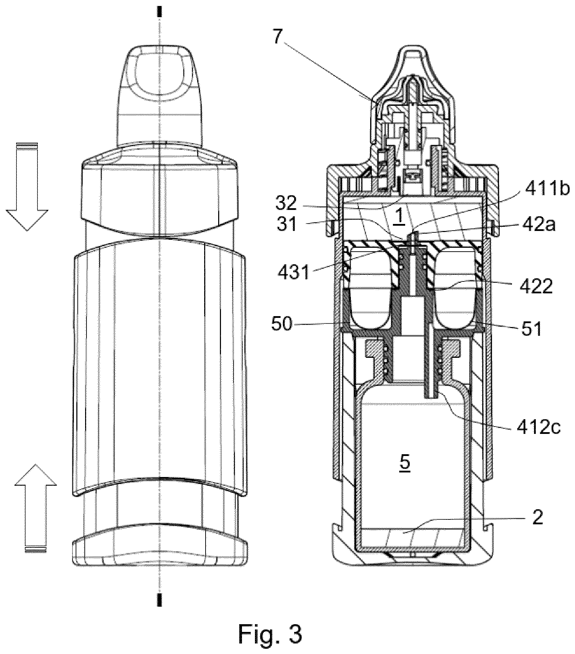

In this original state of the device 100, before the first use thereof, the first ingredient 1 is confined in the enclosed space 3, kept from the outside. The enclosed space 3 is formed by the casing 11 surrounding same and by the plunger head 43, wherein the first barrier 31 prevents fluid communication between the enclosed space 3 and the container 4 which would be established through a central passage 431 (see Figure 3) provided to the plunger head 43 and the fluid communication channel 411 which the plunger rod 42 would provide; and the second barrier 32 prevents fluid communication between the enclosed space 3 and the dispensing group 7, which the tube 113 would provide.

In this original state of the device 100, before the first use thereof, the second ingredient 2 is stored in the container 4, separated from the first ingredient 1 and kept from the outside by the first barrier 31 and by the membrane 51 of the chamber 50, which communicates with the inside of the container 4 through the respiration channel 412, enclosed in suitable environmental conditions established by a gas 5, for example, sterile air.

The support 12 bearing the container 4 is inserted in a displaceable manner with respect to the casing 11 in a loading direction that approaches the enclosed space 3. The support 12 and the casing 11 are both provided with complementary retention means, not identified in the drawings, prepared to prevent or at least hinder the unwanted removal of the support 12 from the casing 11. Known means which can perform this function between parts with relative movement are those working with projections in one part which engage with recesses in the other part by elastic reaction, or vice versa. The support 12 and the casing 11 are both provided with complementary stop means 121 and 111 to limit displacement stroke A of the support 12, and thereby of the container 4 and the associated plunger rod 42, in the loading direction.

The plunger rod 42 and the plunger head 43 are coupled to one another with a gap Z in the loading direction (see Figure 2).

The casing 11 has on one side a window 114 through which it is possible to glimpse the outer surface of the support 12 covering same. Said outer surface has a visible identifier 122, for example, formed by an area with a color that contrasts with the surrounding area. This identifier 122 is visible through the window 114 of the casing 11 in the original state of the device 100.

Based on this original state of the device 100 shown in Figure 2, an operation sequence of the device 100 is explained below, emphasizing other features of the device 100 yet to be described, where necessary.

### Combination of the first and second ingredients

Figures 2 to 4 show the device of Figure 1 in a loading sequence to obtain a substance to be dispensed, which is a combination of two ingredients, in which each figure shows the device externally and according to a plane of section.

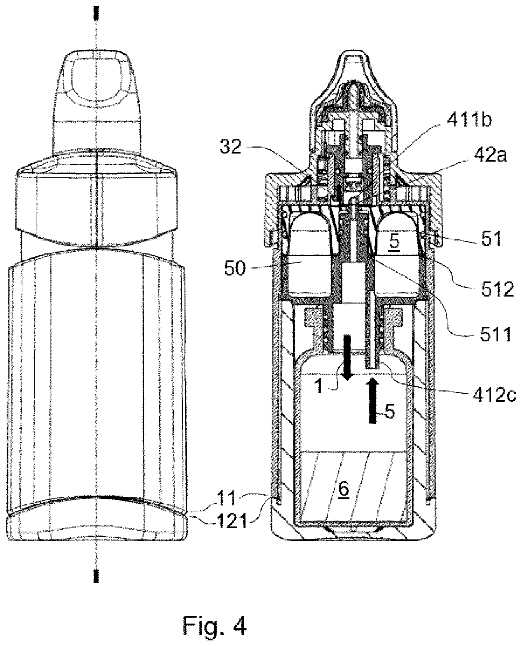

Based on the original state shown in Figure 2, the support 12 and/or the casing 11 are actuated to cause the container 4 and the enclosed space 3 to approach one another. This maneuver is indicated with the arrows of Figure 2, such that:
- In a first phase of displacement stroke A of the container 4 with respect to the casing 11 in the loading direction towards the enclosed space 3, the plunger rod 42 is displaced towards the enclosed space 3 without this displacement being transmitted to the plunger head 43, as a result of the aforementioned gap Z existing between these components.
   The distal end 42a of the plunger rod 42 is particularly configured in correspondence with the gap Z so that, through the passage 431 of the plunger head 43, it reaches the first barrier 31 and opens it, in this specific example by breaking the tear-off seal constituting said first barrier 31, and establishes fluid communication between the enclosed space 3 and the container 4 through the fluid communication channel 411, which situation is illustrated in Figure 3, with the first phase of stroke A thus being completed.
   In the specific exemplary device 100, in an original state of the device 100 before the first use thereof, the first barrier 31 is applied on the face of the plunger head 43 facing the enclosed space 3, specifically covering the passage 431 of the plunger head 43. With the first barrier 31 having a discoidal shape in this case, the barrier 31 is secured at its perimeter area to the edge of the mentioned passage 431 of the plunger head 43.
- In a second phase of displacement stroke A of the container 4 with respect to the casing 11 in the loading direction towards the enclosed space 3, the plunger rod 42 drives the plunger head 43 in movement which reduces the volume of the enclosed space 3 compressing the first ingredient 1, driving it towards the container 4 for the combination thereof inside the container 4 with the second ingredient 2, through the fluid communication channel 411, which situation is illustrated in Figure 4, and obtaining the substance 6 to dispensed, in the example a reconstituted lyophilisate, with the second and last phase of stroke A being completed by interference between the stops 121 and 111 of the support 12 and of the casing 11, respectively.
   The skilled person will understand that the container 4 will be sized to contain the substance 6 resulting from the combination of the first and second ingredients.
   For the plunger rod 42 to drive the plunger head 43 during its displacement, in the specific exemplary device 100, the annular step 422 of the plunger rod 42 is sized to contact and thrust the plunger head 43 through a formation configured in the latter to achieve precise contact and receive this thrust.

It should be noted that, in the specific device 100 which exemplifies the invention, this annular step 422 serving to thrust the plunger head 43 is the same one on which the inner perimeter area 511 of the membrane 51 is fixed.

Figure 4 also shows other effects produced upon completing the second and last phase of stroke A.

Firstly, the distal end 42a of the plunger rod 42 reaches the second barrier 32 and now opens same, in this specific example by breaking another tear-off seal constituting said second barrier 32, now establishing fluid communication between the container 4, which now contains the substance 6, and the dispensing group 7.

In the specific exemplary device 100, in an original state of the device 100 before the first use thereof, the second barrier 32 is applied in the mouth of a tubular drive body 73 of the dispensing group 7, described in greater detail below, fitted in the tube 113 of the casing 11. With the second barrier 32 having a discoidal shape in this case, the barrier 32 is secured at its perimeter area to the edge of an inlet mouth of the mentioned drive body 73.

Secondly, when the first ingredient 1 is introduced in the container 4, the gas 5 which is dislodged from the container 4 is insufflated into the chamber 50, expanding it due to the deformation of the membrane 51.

The skilled person will understand that the size of the membrane 51 will be selected to provide the chamber 50 with sufficient capacity to house this gas 5 dislodged from the container 4.

Accordingly in this case, the change in volume provided to the chamber 50 by the membrane 51 is at least equal to the volume of the first ingredient 1.

In the specific device 100 which exemplifies the invention, the plunger rod 42 and the plunger head 43 have respective rigid parts facing one another and configured to jointly determine a housing inside which the cavity 50 can expand (and then shrink), in this specific example by means of the deformation of the membrane 51.

In this specific example, the aforementioned rigid parts of the plunger 41 come into contact with one another, flattening both the inner perimeter area 511 and the outer perimeter area 512 of the membrane 51 which are interposed in said rigid parts.

To inform the user that the combination of the first and second ingredients 1 and 2 has occurred, and also that the second barrier 32 has opened in the specific example, the visible area of the outer surface of the support 12 which can be glimpsed through the window 114 of the casing 11 is an area surrounding the area of the identifier 122 which is an area with a color that contrasts with the color of the identifier 122. That is, the window 114 shows a change in color.

### Dispensing a dose of the substance

In the example, the container 4 which contains the substance 6 obtained from the combination of the first and second ingredients 1 and 2 is rigid and therefore not suitable for dispensing the substance 6 by exerting external pressure on the container. In any case, it is contemplated that the casing 11 and/or the support 12 bearing the container 4 ensure that no external pressure can be exerted on the device 100 intended for compressing the container 4 in the case where it is not completely rigid.

The absence of a piston, a plunger or a certain moving part inside the container, or linked to the container, which compresses the substance 6 to eject it out of the container, requires not only equipping the device 100 with a dispensing group 7 for dispensing the substance 6, but also requires means to enable dislodging the substance 6 from the container 4 without air intake from the outside to replace the volume of the substance 6 dislodged from same.

The dispensing group 7 can be of several types without this affecting the practice of the invention.

However, it is conceivable for the dispensing groups that will be operatively coupled to the casing 11 of the device to have in common a push button or equivalent for the actuation thereof with relative movement with respect to the casing 11.

If the mentioned relative movement is in the same direction as the loading direction, the device must be provided with means for locking the movement of the push button with respect to the casing of the device when the user handles it to combine the first and second ingredients, which requires displacing the support 12 with respect to the casing 11, as explained.

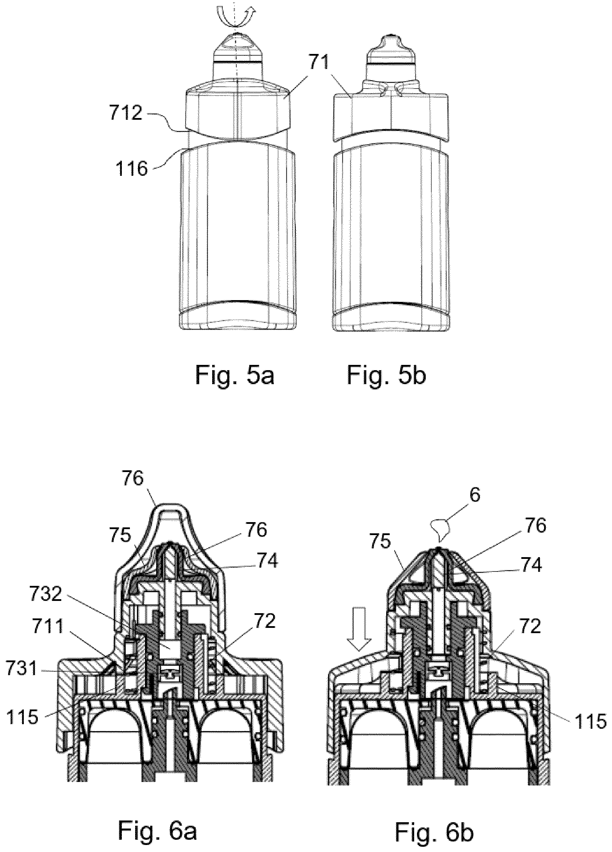

The locking solution implemented in the specific device 100 which exemplifies the invention is based on linking the casing 11 and the push button 71 such as to give rise to the possibility of them rotating with respect to one another, with the push button 71, in the specific exemplary case, being able to adopt a locking position and an unlocking position shown in Figures 5a, 6a and 5b, 6b, respectively, with respect to the casing 11.

Figures 5a and 5b show the device 100 externally once the substance 6 is obtained. In this device 100, the push button 71 is assembled such that it can pivot about the tube 113 of the casing 11, which tube is concealed in these Figures 5a and 5b.

As shown in Figures 6a and 6b, the push button 71 internally has lugs 711 projecting, in the coupling position of the push button 71, towards the closure wall 112 of the casing 11; and a track 115 on which the lugs 711 of the push button 71 can slide is raised in said closure wall 112 of the casing. However, the track is discontinuous, having recesses intended for receiving the fitting of the lugs 711 of the push button 71 when the angular position thereof with respect to the casing 11 causes the lugs 711 to coincide with the recesses of the track 115. When the lugs 711 of the push button 71 rest on the track 115, the push button 71 is in a locking position, shown in Figures 5a and 6a; whereas when the lugs 711 coincide with the recesses of the track 115, the push button 71 is in an unlocking position, shown in Figures 5b and 6b.

In the unlocking position, the push button 71 will travel towards the casing 11 which will promote the expulsion of a dose of the substance 6. In the example, elastic means 72 tend to arrange the push button 71 in a raised position even when the lugs 711 coincide with the recesses of the track 115 in the unlocking position of the push button 71. Elastic fasteners, not identified in the drawings, prevent or at least hinder the accidental separation of the push button 71.

Since these parts intended for cooperating with one another to lock and unlock the push button 71 are not visible from the outside, in the exemplary device 100 the push button 71 has a flared shape and its skirt is finished off with a border 712 which follows a non-straight profile, in the example a slightly wavy profile which in a whole turn along the border has two concave sectors and two curved-concave sectors; whereas the casing 11 is provided laterally with a molding 116 that also alternates concave sectors with curved-concave sectors, in correspondence with those of the push button 71. As Figure 5a attempts to illustrate, when the concave sectors of the skirt of the push button 71 do not match with the concave sectors of the molding of the housing 11, it is a visual indication to the user that the push button 71 is in the locking position.

In the specific device 100 which exemplifies the invention, the dispensing group 7 comprises, in addition to the push button 71, a drive body 73 adapted to be fitted in the tube 113 of the casing 11. The drive body 73 is tubular, determining a passageway having assembled therein a check valve 731. This check valve 731 in the passageway separates a space upstream of the check valve, in permanent fluid communication with the container 4, from a space downstream of the check valve, which can be flooded with a volume of the substance the passage of which is allowed by the check valve and which has a small cavity 732 the volume of which will be abruptly reduced upon actuating the push button 71 in a magnitude equivalent to that of a dose of the substance 6 that will be dispensed through a dispensing needle 74.

For the purpose of forming droplets, a nozzle 75 is coupled on the dispensing needle 74 which serves as a type of elastomeric nipple 76 which is snap-fitted between the dispensing needle 74 and the nozzle 75.

The device 100 can be equipped with a cap 8 to protect the nozzle 75 when the device 100 is not in use.

The successive dispensing of doses of the substance 6 involves dislodging from the container 4 respective equivalent volumes of said substance 6, which volume will be successively occupied by the gas 5 previously accumulated in the leak-tight chamber 50 and with decreasing capacity due to the effect of the membrane 51, which will gradually collapse to adopt a shape similar to that which it adopted with the device 100 in its original state, prior to the combination of the first and second ingredients 1 and 2.

Accordingly, the gas 5 will flow through the respiration channel 412 in the direction opposite the direction it followed when it was dislodged from the container 4.

The invention conceives assembling the device 100 such that the membrane 51 is originally collapsed, with the cavity 50 being substantially empty of gas. However, other variants of the invention are possible and it is also conceived, for example, to assemble the device 100 such that the membrane 51 is slightly inflated in accordance with a gas atmosphere inside the container 4 at a pressure slightly higher than atmospheric pressure. The conditions for assembling the device 100 will determine the conditions in which the membrane 51 will be when all the substance 6 has been dispensed.

Taking advantage of the fact that the substance 6 is for ophthalmological use and that it is therefore to be expected that doses of the substance 6 are dispensed while the device 100 is upside down, so that the substance 6 is deposited by gravity in a target eye, in the specific device 100 which exemplifies the invention the mentioned respiration channel 412 has a prolongation 412c the purpose of which is to locate the end of this respiration channel 412 that opens into the container 4 above the free surface of the substance 6 when the device 100 is turned upside down.

## Claims

1. A device (100) for combining ingredients of a substance (6) and for dispensing it in a controlled manner, wherein the device (100) comprises an enclosed space (3) of variable volume which contains, under leak-tight conditions, a first ingredient (1) in a fluid state; a container (4) which contains, under leak-tight conditions, a second ingredient (2) in a gas atmosphere (5) and which is linked to a plunger (41) operating on the enclosed space (3); and a liquid dose dispensing group (7) that can be actuated, the device (100) being **characterized in that** it further comprises a first and a second barrier (31, 32) which, in an original state of the device (100) before the first use thereof, prevent fluid communication between the enclosed space (3), the container (4), and the dispensing group (7), the plunger (41) being configured such that, in displacement stroke A of the container (4) with respect to the enclosed space, in a loading direction that approaches the enclosed space (3), the following actions are sequentially triggered:
- opening of the first barrier (31), enabling fluid communication between the enclosed space (3) and the container (4),
- driving of the first ingredient (1) to the container (4), for the combination thereof with the second ingredient (2) inside the container (4) obtaining the substance (6), and
- opening of the second barrier (32), enabling fluid communication between the container (4) and the dispensing group (7),
with the device (100) further being provided with a cavity (50) adapted for insufflating into same the gas (5) which is dislodged from the container (4) by introducing the first ingredient into the container (4) and for exhaling this gas (5) back into the container (4) as doses of the substance (6) are dispensed as a result of the actuation of the dispensing group (7).

2. The device (100) according to claim 1, **characterized in that** the plunger (41) comprises a fluid communication channel (411) with a first end (411a) that opens into the container (4) and with a second end (411b) that opens into the enclosed space (3) in a first phase of stroke A and into the dispensing group (7) in a second phase of stroke A.

3. The device (100) according to claim 1 or 2, **characterized in that** the plunger (41) comprises a respiration channel (412) with a first end (412a) that opens into the container (4) and with a second end (412b) that opens into the cavity (50).

4. The device (100) according to claims 2 and 3, **characterized in that** the plunger (41) comprises a plunger rod (42) and a plunger head (43), wherein the plunger rod (42) is firmly connected to a mouth of the container (4) and supports the fluid communication channel (411) and the respiration channel (412); and wherein the plunger head (43) has a passage (431) for the plunger rod (42) therethrough; the plunger rod (42) and the plunger head (43) being originally coupled to one another with a gap (Z) in the loading direction such that sequentially in displacement stroke A of the container (4), the first barrier (31) opens due to the thrusting of the distal end (42a) of the plunger rod (42) through the passage (431) of the plunger head (43), the enclosed space (3) shrinks due to the displacement of the plunger head (43), driven in movement by the plunger rod (42), and the second barrier (32) opens due to the thrusting of the distal end (42a) of the plunger rod (42).

5. The device (100) according to any one of the preceding claims, **characterized in that** the cavity (50) is a cavity with the capacity to expand and shrink varying its volume by an amount at least equal to the volume of the first ingredient (1).

6. The device (100) according to the preceding claim, **characterized in that** at least one wall of the cavity (50) is a flexible wall which provides the cavity (50) with the possibility of inflating and deflating when the gas (5) dislodged from the container (4) is insufflated into same and when the gas (5) is insufflated into the container (4), respectively.

7. The device (100) according to the preceding claim, **characterized in that** the flexible wall of the cavity (50) is formed by a single membrane (51) secured in a leak-tight manner to the plunger rod (42).

8. The device (100) according to the preceding claim, **characterized in that** the plunger rod (42) comprises a rigid overhang (421) with a bottom and a side wall; and **in that** the membrane (51) has a central passage for the plunger rod (42), with there being determined in the membrane (51) respective inner perimeter area (511) and outer perimeter area (512) secured to an annular step formation (422) of the plunger rod (42) and to the border of the side wall of the mentioned overhang (421), respectively.

9. The device (100) according to any one of claims 2 to 7, **characterized in that** the plunger rod (42) and the plunger head (43) comprise respective rigid parts facing one another and configured to jointly determine a housing inside which the cavity (50) can expand and shrink.

10. The device (100) according to any one of the preceding claims, **characterized in that** it comprises end of stroke A stops.

11. The device (100) according to any one of the preceding claims, **characterized in that** the second ingredient (2) is a lyophilisate; **in that** the first ingredient (1) is a serum for reconstituting the lyophilisate; and **in that** the substance (6) is a drug substance.

12. The device (100) according to the preceding claim, **characterized in that** the container (4) is a rigid container formed by a vial suitable for being the same one used during preparation of the lyophilisate.

13. The device (100) according to any one of the preceding claims, **characterized in that** the first and second barriers (31, 32) are respective breakable seals.

14. The device (100) according to any one of the preceding claims, **characterized in that** it comprises an outer casing (11) and a support (12) bearing the container (4), wherein the casing (11) receives at one service end the fitting of the support (12) bearing the container (4), and wherein the dispensing group (7) comprises at least one push button (71) that can be actuated to promote the dispensing of a dose of the substance (6) that is operatively coupled to the casing (11) at one end of the casing (11) opposite the service end, with the push button (71) being able to adopt at least two angular positions with respect to the casing (11), one of which is a locking position and the other an unlocking position, with the push button (71) being able to be actuated only towards the casing (11) and in the loading direction to promote the dispensing of a dose of the substance (6) when it adopts the unlocking position.

15. A method for combining ingredients of a substance (6) and dispensing it in a controlled manner, wherein the method comprises, with one and the same device:
- enabling fluid communication between an enclosed space (3) of variable volume which contains in an original state a first ingredient (1) in a fluid state and a container (4) which contains in an original state a second ingredient (2) in a gas atmosphere (5);
- transferring the first ingredient (2) into the container (4) to obtain the substance (6), where the substance results from the combination of the first and second ingredients (1, 2) inside the container (4); and
- enabling fluid communication between the container (4) and a liquid dose dispensing group (7) that can be actuated to dispense doses of the substance (6),
the method further comprising:
- insufflating gas (5) dislodged from the container (4) when the first ingredient (1) is transferred into a cavity (50) configured to exhale gas (5) from the cavity (50) back into the container (4) when doses of the substance (6) are dispensed as a result of the actuation of the dispensing group (7).
